(19) Europäisches Patentamt
European Patent Office
Office européen des brevets

(11) Numéro de publication: **0 183 590**
**B1**

# (12) FASCICULE DE BREVET EUROPÉEN

(45) Date de publication du fascicule du brevet: **18.05.88**

(51) Int. Cl.⁴: **C 07 C 19/08**, C 07 C 17/26

(21) Numéro de dépôt: **85402141.7**

(22) Date de dépôt: **06.11.85**

(54) **Hydrocarbures perchlorofluorés et leur procédé de préparation.**

(30) Priorité: **13.11.84 FR 8417278**

(43) Date de publication de la demande: **04.06.86 Bulletin 86/23**

(45) Mention de la délivrance du brevet: **18.05.88 Bulletin 88/20**

(84) Etats contractants désignés: **AT BE CH DE FR GB IT LI LU NL SE**

(56) Documents cité: **EP-A-0 140 385**

(73) Titulaire: **ATOCHEM, 4 & 8, Cours Michelet La Défense 10, F-92800 Puteaux (FR)**

(72) Inventeur: **Boutevin, Bernard, Les Terres Blanches 1, rue Anselme Mathieu, F-34000 Montpellier (FR)**
Inventeur: **Hervaud, Yves, 174, avenue de Lodève, F-34000 Montpellier (FR)**

(74) Mandataire: **Leboulenger, Jean, ATOCHEM Département Propriété Industrielle, F-92091 Paris la Défense 10 Cédex 42 (FR)**

EP 0 183 590 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention concerne le domaine des hydrocarbures perhalogénés et a plus particulièrement pour objet de nouveaux hydrocarbures perchlorofluorés et leur procédé de préparation.

Dans la série des hydrocarbures perhalogénés, notamment perchlorofluorés, les premiers termes sont connus depuis longtemps et sont largement utilisés dans de nombreux domaines (conditionnement aérosol, réfrigération, fabrication de mousses plastiques, nettoyage, etc...).

Des hydrocarbures perchlorofluorés plus lourds, par exemple ceux constitués par des télomères du chlorotrifluoroéthylène ou du tétrafluoroéthylène avec le tétrachlorure de carbone, sont connus (voir par exemple Tetrahedron Letters vol. 1973, fasc. 12, 887; Eur. Polym. J. 1976, 12, 219; J. Am. Chem. Soc. 1961, 83, 3425; brevets EP 93580, GB 1 007 542 et GB 1 127 045), ces télomères sont de précieux intermédiaires pour la synthèse de composés chlorofluorés fonctionnels utilisables pour la préparation de polymères à propriétés hydrophobes et oléophobes. La transformation de ces télomères en acides et alcools chlorofluorés a notamment été étudiée par BOUTEVIN et al dans Tetrahedron Letters vol. 1974, fasc. 12, 939; Eur. Polym. J. 1976, 12, 231 et Makrom. Chem. 1981, 182, 2927.

Il a maintenant été trouvé qu'on peut obtenir à partir de ces télomères de nouveaux hydrocarbures perchlorofluorés qui répondent à la formule générale:

$$Cl\text{-}(\text{-}CFX\text{-}CF_2)_m\text{-}CCl_2\text{-}(\text{-}CF_2\text{-}CFY)_n\text{-}Cl \quad (I)$$

dans laquelle les symboles X et Y, identiques ou différents, représentent chacun un atome de chlore ou de fluor, m et n sont des nombres entiers, identiques ou différents, allant de 1 à 5, mais de préférence égaux à 1.

Les composés de formule (I) selon l'invention peuvent être obtenus en faisant réagir le chlorotrifluoroéthylène ou le tétrafluoroéthylène sur un télomère de formule:

$$Cl_3\text{-}(\text{-}CF_2\text{-}CFX)_m\text{-}Cl \quad (II)$$

dans laquelle X et m ont les mêmes significations que précédemment, dans un solvant organique polaire autre qu'un alcool à une température d'au moins 120° C et en présence de cuivre et/ou d'un sel de cuivre comme catalyseur.

Comme exemples de solvants polaires, on peut citer plus particulièrement les nitriles comme l'acétonitrile, le butyronitrile ou l'isobutyronitrile, les éthers comme le tétrahydrofurane et les amides comme le diméthylformamide. La quantité de solvant peut varier dans de larges limites. Cependant, afin de limiter la pression dans le réacteur, il est avantageux de mettre en oeuvre une proportion de 0,5 à 5 et de préférence environ 2 moles de solvant par mole de monomère (chlorotrifluoroéthylène ou tétrafluoroéthylène).

Dans le catalyseur, le cuivre peut être à l'état Cu°, $Cu^{+}$ et/ou $Cu^{++}$. Bien qu'on préfère utiliser le cuivre sous forme de chlorure, la nature de l'anion est sans grande importance. La quantité de cuivre et/ou de sel de cuivre à utiliser pour une mole de monomère peut varier de $5.10^{-3}$ a $5.10^{-2}$ mole et est de préférence voisine de $1.10^{-2}$ mole. La valeur de n est d'autant plus petite que la quantité de catalyseur est élevée.

La réaction doit être effectuée à une température d'au moins 120° C. Bien qu'on préfère opérer à environ 140° C pour limiter la pression dans le réacteur, il est possible d'opérer à des températures plus élevées pouvant aller jusqu'à environ 180° C.

Le rapport molaire du monomère au télomère peut varier entre 1 et 15 en fonction du composé (I) désiré, la valeur de n augmentant dans le même sens.

Les composés de formule (I) dans laquelle X et Y sont identiques peuvent aussi être obtenus en faisant réagir le chlorotrifluoroéthylène ou le tétrafluoroéthylène sur le tétrachlorure de carbone dans les mêmes conditions que précédemment mais avec un rapport molaire $CFX{=}CF_2/CCl_4$ au moins égal à 2 et de préférence compris entre 2 et 20, la valeur de m + n augmentant dans le même sens. La valeur de m + n est d'autant plus petite que la quantité de catalyseur est élevée.

Les composés de formule (I) selon l'invention sont de précieux intermédiaires pour la synthèse de composés chlorofluorés fonctionnels utilisables comme agents tensioactifs ou comme matières premières pour la préparation de polymères à propriétés hydrophobes et oléophobes. Comme les télomères dont ils dérivent, les composés selon l'invention peuvent notamment être facilement transformés en acides, puis en alcools par des méthodes connues en soi telles que celles décrites par BOUTEVIN et al dans les références précitées.

Les exemples suivants illustrent l'invention, sans la limiter. Les spectres de $RMN^{19}F$ ont été effectués sur un appareil VARIAN E.M. 390 avec une fréquence égale à 84,67 MHz en utilisant $CFCl_3$ comme référence. Les spectres de masse ont été réalisés avec un appareil CEC 211 10C (double focalisation) muni d'une source à ionisation (100 µA, 70 eV) et d'un système d'introduction directe. On indique les valeurs m/e et, entre parenthèses, le pourcentage relatif à chaque pic par rapport au pic de base.

**Exemple 1**

a) Dans un autoclave à cuve en acier vitrifié (réacteur PFAUDLER) de 4,5 litres muni d'un dispositif de chauffage par circulation d'huile, d'un système d'agitation avec variateur de

vitesse et d'une vanne pour l'introduction de gaz, on charge 1,54 kg (10 moles) de tétrachlorure de carbone, puis 13,45 g (0,1 mole) de chlorure cuivrique dissous dans un demi litre d'acétonitrile. Après fermeture du réacteur, on introduit 1,165 kg (10 moles) de chlorotrifluoroéthylène, puis on chauffe le mélange à 120° C pendant 24 heures sous agitation à 200 tours/minute. Après lavage à l'eau acidulée pour éliminer le cuivre et distillation sous vide (20 torr), on obtient 2,57 kg du télomère $CFCl_2$-$CF_2$-$CCl_3$ (Eb 15 torrs: 47° C)

b) Dans le même réacteur que ci-dessus, on charge 2,705 kg (10 moles) de télomère $CFCl_2$-$CF_2$-$CCl_3$ et 13,5 g (0,1 mole) de chlorure cuivrique en solution dans un litre d'acétonitrile. On ferme le réacteur et introduit 1,165 kg (10 moles) de chlorotrifluoréthylène, puis on chauffe le mélange à 140° C pendant 48 heures sous agitation à 200 tours/minute. Par distillation sous vide, on obtient avec un rendement de 60 % l'hexachloro-1,1,3,3,5,5 hexafluoro-1,2,2,4,4,5 pentane qui bout à 97° C sous 15 torrs.

Analyse:

| | C % | Cl % | F % |
|---|---|---|---|
| Calculé pour $C_5Cl_6F_6$ | 15,50 | 55,04 | 29,46 |
| Trouvé | 15,92 | 55,78 | 28,07 |

Spectre RMN$^{19}$F:

Massif (2F) centré à $64,33.10^{-6}$
Massif (4F) centré à $101.10^{-6}$

Spectre de masse:

357(2,5); 355(4); 353(6); 351(5); 349(4); 301(2,5); 299(5); 297(11); 295(7,5); 252(2,5); 250(5); 248(4); 239(15); 237(40); 235(67); 233(60); 221(18); 219(32); 217(35); 153(18); 151(25); 105(18); 103(68); 101(100); 85(28)

**Exemple 2**

Dans un tube de CARIUS en pyrex (épaisseur: 2 mm, longueur: 260 mm, diamètre: 23 mm), on mélange 99 mg de chlorure cuivreux, 38,7 g (0,1 mole) du télomère $CCl_3$-$(CF_2CFCl)_2$-Cl décrit par Boutevin et Pietrasanta (Eur. Polym. J. 1976, 12, 219) et 10 ml d'acétonitrile. On refroidit le tube dans un mélange acétone-carboglace, puis on y introduit 11,65 g (0,1 mole) de chlorotrifluoroéthylène et le scelle. On chauffe le tube scellé pendant 16 heures à 180° C dans un autoclave agité par balancement.

Après ouverture du tube, lavage du produit réactionnel avec une solution diluée d'HCl pour éliminer le cuivre et extraction à l'éther, on distille sous vide et obtient ainsi 13,6 g d'heptachloro-1,1,3,3,5,7,7 nonafluoro-1,2,2,4,4,5,6,6,7 heptane qui bout à 97° C sous 0,1 torr. Teneur en chlore: 49,01 % (calculé pour $C_7Cl_7P_9$: 49,35 %).

Spectre RMN$^{19}$F:

massif (1F) centré à $125.10^{-6}$
massif (2F) centré à $108,8.10^{-6}$
massif (4P) entre 97 et $106.10^{-6}$
massif (1F) centré à $67.10^{-6}$
massif (1F) centré à $64,7.10^{-6}$

Spectre de Masse:

473(0,4); 471(0,8); 469(1,5); 467(2); 465(1); 417(1); 415(4); 413(6); 411(4); 357(1); 355(4); 353(8); 351(12); 349(8); 399(0,5); 297(4); 295(3); 283(1); 281(1); 279(4); 271(3); 269(9); 267(10); 253(1); 251(5); 249(4); 239(4); 237(16); 235(26); 233(22); 185(1); 183(1); 179(12); 167(4); 165(17); 163(16); 155(6); 153(18); 151(26); 105(18); 103(70); 101(100); 87(22); 85(62); 69(20).

**Exemple 3**

Dans l'autoclave décrit à l'exemple 1, on charge 1,54 kg (10 moles) de tétrachlorure de carbone, puis 26,9 g (0,2 mole) de chlorure cuivrique dissous dans 1,5 l d'acétonitrile. On introduit ensuite 2,330 kg (20 moles) de chlorotrifluoroéthylène. Après 24 heures de chauffe à 140° C, sous agitation à 200 tours/minute on obtient 3,280 kg d'un mélange des produits $CFCl_2$ - $CF_2$ - $CCl_3$ et $CFCl_2$ - $CF_2$ - $CCl_2$ - $CF_2$ - $CFCl_2$ dans les proportions respectives 70/30 en poids. Après 48 heures de chauffe à 140° C, on obtient 3,400 kg du mélange dans les proportions 50/50 en poids.

Les produits sont identifiés comme dans les exemples précédents.

**Revendications** pour les Etats contractants BE, CH, DE, FR, GB, IT, LI, LU, NL, SE

1. Hydrocarbures perchlorofluorés de formule générale:

$$Cl -(-CFX-CF_2)_m - CCl_2 -(-CF_2-CFY)_n - Cl$$

dans laquelle les symboles X et Y, identiques ou différents, représentent chacun un atome de chlore ou de fluor, m et n sont des nombres entiers, identiques ou différents, allant de 1 à 5.

2. Hydrocarbures perchlorofluorés selon la revendication 1, dans lesquels chacun des nombres m et n est égal à 1.

3. Hydrocarbures perchlorofluorés selon la revendication 1 ou 2, dans lesquels X et Y représentent chacun un atome de chlore.

4. Procédé de préparation des hydrocarbures perchlorofluorés selon la revendication 1,

caractérisé en ce que l'on fait réagir le chlorotrifluoroéthylène ou le tétrafluoroéthylène sur un télomère de formule:

$Cl_3C$ -(-$CF_2$-CFX$)_m$ - Cl

dans laquelle X et m ont les mêmes significations que dans la revendication 1, dans un solvant organique polaire à une température d'au moins 120°C et en présence de cuivre et/ou d'un sel de cuivre comme catalyseur.

5. Procédé selon la revendication 4, dans lequel on fait réagir le chlorotrifluoroéthylène sur le pentachloro-1,1,1,3,3 trifluoro-2,2,3 propane.

6. Procédé de préparation des hydrocarbures perchlorofluorés selon la revendication 1, dans lesquels X et Y sont identiques, caractérisé en ce que l'on fait réagir en l'absence de fer le chlorotrifluoroéthylène ou le tétrafluoroéthylène sur le tétrachlorure de carbone dans un solvant organique polaire à une température d'au moins 120°C et en présence de cuivre et/ou d'un sel de cuivre comme catalyseur, le rapport molaire du chlorotrifluoroéthylène ou du tétrafluoroéthylène sur le tétrachlorure de carbone étant au moins égal à 2.

7. Procédé selon la revendication 6, dans lequel on fait réagir le chlorotrifluoroéthylène sur le tétrachlorure de carbone.

8. Procédé selon l'une des revendications 4 à 7, dans lequel on utilise de $5.10^{-3}$ à $5.10^{-2}$ mole de catalyseur par mole de chlorotrifluoroéthylène ou de tétrafluoroéthylène.

9. Procédé selon l'une des revendications 4 à 8, dans lequel le solvant polaire est un nitrile, un éther ou un amide.

10. Procédé selon l'une des revendications 4 à 9, dans lequel le catalyseur est un chlorure de cuivre.

11. Procédé selon l'une des revendications 4 à 10, dans lequel le solvant polaire est l'acétonitrile.

**Revendications** pour l'Etat contractant AT

1. Procédé de préparation d'hydrocarbures perchlorofluorés de formule générale:

Cl -(-CFX-$CF_2)_m$ - $CCl_2$ -(-$CF_2$-CFY$)_n$ - Cl

dans laquelle les symboles X et Y, identiques ou différents, représentent chacun un atome de chlore ou de fluor, m et n sont des nombres entiers, identiques ou différents, allant de 1 à 5 caractérisés en ce que l'on fait réagir en l'absence de fer le chlorotrifluoroéthylène ou le tétrafluoroéthylène sur le tétrachlorure de carbone ou un télomère de formule:

$Cl_3C$ -(-$CF_2$-CFX$)_m$ - Cl

dans laquelle X et m ont les mêmes significations que ci-dessus, dans un solvant organique polaire à une température d'au moins 120°C et en présence de cuivre et/ou d'un sel de cuivre comme catalyseur, le rapport molaire du chlorotrifluoroéthylène ou du tétrafluoroéthylène au tétrachlorure de carbone étant au moins égal à 2.

2. Procédé selon la revendication 1, dans lequel on fait réagir le chlorotrifluoroéthylène sur le pentachloro-1,1,1,3,3 trifluoro-2,2,3 propane.

3. Procédé selon la revendication 1, dans lequel on fait réagir le chlorotrifluoroéthylène sur le tétrachlorure de carbone.

4. Procédé selon l'une des revendications 1 à 3, dans lequel on utilise de $5.10^{-3}$ à $5.10^{-2}$ mole de catalyseur par mole de chlorotrifluoroéthylène ou de tétrafluoroéthylène.

5. Procédé selon l'une des revendications 1 à 4, dans lequel le solvant polaire est un nitrile, un éther ou un amide.

6. Procédé selon l'une des revendications 1 à 5, dans lequel le catalyseur est un chlorure de cuivre.

7. Procédé selon l'une des revendications 1 à 6, dans lequel le solvant polaire est l'acétonitrile.

**Patentansprüche** für die Vertragsstaaten BE, CH, DE, FR, GB, IT, LI, LU, NL, SE

1. Perchlorfluorierte Kohlenwasserstoffe der allgemeinen Formel:

Cl -(-CFX-$CF_2)_m$ - $CCl_2$ -(-$CF_2$-CFY$)_n$ - Cl

in der die Reste X und Y identisch oder verschieden sind und jeweils ein Chloratom oder Fluoratom darstellen, m und n identisch oder verschieden sind und ganze Zahlen zwischen 1 und 5 darstellen.

2. Perchlorfluorierte Kohlenwasserstoffe nach Anspruch 1, bei denen jede Zahl m und n gleich 1 ist.

3. Perchlorfluorierte Kohlenwasserstoffe nach Anspruch 1 oder 2, bei denen X und Y jeweils ein Chloratom darstellen.

4. Verfahren zur Herstellung der perchlorfluorierten Kohlenwasserstoffe gemäß Anspruch 1, dadurch gekennzeichnet, daß man Chlortrifluorethylen oder Tetrafluorethylen mit einem Telomeren der Formel:

$Cl_3C$ -(-$CF_2$-CFX$)_m$ - Cl

in der X und m die gleiche Bedeutung haben wie in Anspruch 1, in einem polaren organischen Solvens bei einer Temperatur von wenigstens 120°C und in Gegenwart von Kupfer und/oder eines Kupfersalzes als Katalysator reagieren läßt.

5. Verfahren nach Anspruch 4, bei dem man Chlortrifluorethylen mit 1,1,1,3,3-Pentachlor-2,2,3-Trifluorpropan reagieren läßt.

6. Verfahren zur Herstellung von perchlorfluorierten Kohlenwasserstoffen gemäß Anspruch 1, bei denen X und Y identisch sind,

dadurch gekennzeichnet, daß man in Abwesenheit von Eisen Chlortrifluorethylen oder Tetrafluorethylen mit Tetrachlorkohlenstoff in einem polaren organischen Solvens bei einer Temperatur von wenigstens 120°C und in Gegenwart von Kupfer und/oder einem Kupfersalz als Katalysator reagieren läßt, wobei das molare Verhältnis von Chlortrifluorethylen oder Tetrafluorethylen zu Tetrachlorkohlenstoff wenigstens gleich 2 ist.

7. Verfahren nach Anspruch 6, bei dem man Chlortrifluorethylen mit Tetrachlorkohlenstoff reagieren läßt.

8. Verfahren nach einem der Ansprüche 4 bis 7, bei dem man $5.10^{-3}$ bis $5.10^{-2}$ Mol des Katalysators pro Mol Chlortrifluorethylen oder Tetrafluorethylen verwendet.

9. Verfahren nach einem der Ansprüche 4 bis 8, bei dem das polare Solvens ein Nitril, ein Äther oder ein Amid ist.

10. Verfahren nach einem der Ansprüche 4 bis 9, bei dem der Katalysator ein Kupferchlorid ist.

11. Verfahren nach einem der Ansprüche 4 bis 10, bei dem das polare Solvens Acetonitril ist.

**Patentansprüche** für den Vertragsstaat AT

1. Verfahren zur Herstellung von perchlorfluorierten Kohlenwasserstoffen der allgemeinen Formel

$$Cl\text{-}(\text{-}CFX\text{-}CF_2)_m\text{-}CCl_2\text{-}(\text{-}CF_2\text{-}CFY)_n\text{-}Cl$$

in der die Reste X und Y identisch oder verschieden sind und jeweils ein Chloratom oder Fluoratom darstellen, m und n identisch oder verschieden sind und ganze Zahlen zwischen 1 und 5 darstellen, dadurch gekennzeichnet, daß man in Abwesenheit von Eisen Chlortrifluorethylen oder Tetrafluorethylen mit Tetrachlorkohlenstoff oder einem Telomeren der Formel:

$$Cl_3C\text{-}(\text{-}CF_2\text{-}CFX)_m\text{-}Cl$$

in der X und m die gleiche Bedeutung haben wie oben, in einem polaren organischen Solvens bei einer Temperatur von wenigstens 120°C und in Gegenwart von Kupfer und/oder einem Kupfersalz als Katalysator reagieren läßt, wobei das molare Verhältnis von Chlortrifluorethylen oder Tetrafluorethylen zu Tetrachlorkohlenstoff wenigstens gleich 2 ist.

2. Verfahren nach Anspruch 1, bei dem man Chlortrifluorethylen mit 1,1,1,3,3-Pentachlor-2,2,3-trifluorpropan reagieren läßt.

3. Verfahren nach Anspruch 1, bei dem man Chlortrifluorethylen mit Tetrachlorkohlenstoff reagieren läßt.

4. Verfahren nach einem der Ansprüche 1 bis 3, bei dem man $5.10^{-3}$ bis $5.10^{-2}$ Mol des Katalysators pro Mol Chlortrifluorethylen oder Tetrafluorethylen einsetzt.

5. Verfahren nach einem der Ansprüche 1 bis 4, bei dem das polare Solvens ein Nitril, ein Äther oder ein Amid ist.

6. Verfahren nach einem der Ansprüche 1 bis 5, bei dem der Katalysator ein Kupferchlorid ist.

7. Verfahren nach einem der Ansprüche 1 bis 6, bei dem das polare Solvens Acetonitril ist.

**Claims** for the Contracting States BE, CH, DE, FR, GB, IT, LI, LU, NL, SE

1. Perchlorofluorinated hydrocarbons of general formula:

$$Cl\text{-}(\text{-}CFX\text{-}CF_2)_m\text{-}CCL_2\text{-}(\text{-}CF_2\text{-}CFY)_n\text{-}Cl$$

in which each of the symbols X and Y, which may be identical or different, denotes a chlorine or fluorine atom and m and n are identical or different integers, from 1 to 5.

2. Perchlorofluorinated hydrocarbons according to Claim 1, in which each of the numbers m and n is equal to 1.

3. Perchlorofluorinated hydrocarbons according to Claim 1 or 2, in which X and Y each denote a chlorine atom.

4. Process for the preparation of perchlorofluorinated hydrocarbons according to Claim 1, characterized in that chlorotrifluoroethylene or tetrafluoroethylene is reacted with a telomer of formula:

$$Cl_3C\text{-}(\text{-}CF_2\text{-}CFX)_m\text{-}Cl$$

in which X and m have the same meanings as in Claim 1, in an organic polar solvent at a temperature of at least 120°C and in the presence of copper and/or a copper salt as catalyst.

5. Process according to Claim 4, in which chlorotrifluoroethylene is reacted with 1,1,1,3,3,-pentachloro-2,2,3-trifluoropropane.

6. Process for the preparation of perchlorofluorinated hydrocarbons according to Claim 1, in which X and Y are identical, characterized in that chlorotrifluoroethylene or tetrafluoroethylene is reacted with carbon tetrachloride in a polar organic solvent at a temperature of at least 120°C in the absence of iron and in the presence of copper and/or of a copper salt as catalyst, the molar ratio of chlorotrifluoroethylene or of tetrafluoroethylene to carbon tetrachloride being equal to at least 2.

7. Process according to claim 6, in which chlorotrifluoroethylene is reacted with carbon tetrachloride.

8. Process according to one of Claims 4 to 7, in which from $5x10^{-3}$ to $5x10^{-2}$ mole of catalyst per mole of chlorotrifluoroethylene or tetrafluoroethylene is used.

9. Process according to one of Claims 4 to 8, in which the polar solvent is a nitrile, an ether or an amide.

10. Process according to one of Claims 4 to 9, in which the catalyst is a copper chloride.

11. Process according to one of Claims 4 to 10, in which the polar solvent is acetonitrile.

**Claims** for the Contracting State AT.

1. Process for the preparation of perchlorofluorinated hydrocarbons of general formula:

$$Cl\text{-}(\text{-}CFX\text{-}CF_2)_m\text{ - }CCl_2\text{-}(\text{-}CF_2\text{-}CFY)_n\text{ - }Cl$$

in which each of the symbols X and Y, which may be identical or different, denotes a chlorine or fluorine atom, m and n are identical or different integers, from 1 to 5, characterized in that chlorotrifluoroethylene or tetrafluroroethylene is reacted with carbon tetrachloride or a telomer of formula

$$Cl_3C\text{-}(\text{-}CF_2\text{-}CFX)_m\text{ - }Cl$$

in which X and m have the same meaning as above, in a polar organic solvent at a temperature of at least 120° C, in the absence of iron and in the presence of copper and/or of a copper salt as catalyst, the molar ratio of chlorotrifluoroethylene or of tetrafluoroethylene to carbon tetrachloride being equal to at least 2.

2. Process according to Claim 1, in which chlorotrifluoroethylene is reacted with 1,1,1,3,3-pentachloro-2,2,3-trifluoroethylene.

3. Process according to Claims 1, in which chlorotrifluoroethylene is reacted with carbon tetrachloride.

4. Process according to one of Claims 1 to 3, in which from $5x10^{-3}$ to $5x10^{-2}$ mole of catalyst is used per mole of chlorotrifluoroethylene or of tetrafluoroethylene.

5. Process according to one of Claims 1 to 4, in which the polar solvent is a nitrile, an ether or an amide.

6. Process according to one of Claims 1 to 5, in which the catalyst is a copper chloride.

7. Process according to one of Claims 1 to 6, in which the polar solvent is acetonitrile.